# EUROPÄISCHE PATENTANMELDUNG

(11) **EP 4 843 591 A1**
(43) Veröffentlichungstag der Anmeldung: **25.11.2026**
(21) Anmeldenummer: 25177455.0
(22) Anmeldetag: 19.05.2025
(51) Int. Cl.: A61M 5/145

(54) **MEDIZINISCHER EINMALARTIKEL MIT MASCHINENLESBAREM ERKENNUNGSMERKMAL UND MEDIZINISCHE VORRICHTUNG**

(71) Anmelder: B. Braun Melsungen AG, 34212 Melsungen (DE)
(72) Erfinder: KAUBA, Michael, 34277 Fuldabrück (DE); VOGEL, Markus, 37191 Katlenburg-Lindau (DE); SIELEMANN, Martin, 34466 Wolfhagen (DE); OSTERMOELLER, Michael, 36251 Bad Hersfeld (DE)
(74) Vertreter: Tergau & Walkenhorst Intellectual Property GmbH

(57) **Zusammenfassung**

Medizinischer Einmalartikel (2) für eine medizinische Vorrichtung (50), insbesondere Spritzenpumpe (10), wobei der Einmalartikel (2) bevorzugt eine Spritze (6) zur Verwendung in Spritzenpumpen (10) ist, umfassend ein Körperteil (16) und umfassend ein in einem Teilabschnitt (3) des Körperteils (106) angeordnetes maschinenlesbares Erkennungsmerkmal (34) mit wenigstens einem optisch erfassbaren Code, wobei das maschinenlesbare Erkennungsmerkmal (34) ein Merkmal des Einmalartikels (2) ist oder in dem vorbestimmten Teilabschnitt (30) des Einmalartikels (2) lösbar oder unlösbar, insbesondere werkseitig, angebracht ist, und wobei das Erkennungsmerkmal (34) zumindest bereichsweise umfangsseitig am vorbestimmten Teilabschnitt (30) des Körperteils (16) angeordnet ist.

## Beschreibung

### Technisches Gebiet

Die vorliegende Erfindung betrifft einen medizinischen Einmalartikel für eine medizinische Vorrichtung, insbesondere eine Spritze zur Verwendung in einer Spritzenpumpe, eine medizinische Vorrichtung, insbesondere eine Spritzenpumpe, zur automatisierten Verabreichung von Medikamenten an einen Patienten und ein System und ein Verfahren zur erkennungsmerkmalabhängigen, insbesondere personalisierten, Medikamentenverabreichung.

### Hintergrund der Erfindung

Im Stand der Technik sind medizinische Vorrichtungen, insbesondere Spritzenpumpen bekannt, die dazu dienen, in der Therapie eines Patienten eingesetzt zu werden und insbesondere Medikamente an den Patienten automatisiert zu verabreichen. Dabei werden medizinische Einmalartikel, wie beispielsweise Spritzen zur Verwendung in Spritzenpumpen, in die medizinische Vorrichtung eingebracht, die sodann das in dem Einmalartikel, beispielsweise einer Spritze zur Verwendung in Spritzenpumpen, enthaltene Medikament kontrolliert an den Patienten befördert. Dabei wird insbesondere eine in der medizinischen Vorrichtung vorhandene Antriebseinheit verwendet, die durch eine entsprechende Steuereinrichtung angesteuert werden kann.

Beispielsweise kann ein Medikament oder eine andere medizinische Lösung, wie beispielsweise Kochsalzlösung, in einer Spritze zur Verwendung in Spritzenpumpen bereitgestellt werden. Diese Zubehörartikel mit beinhaltendem Medikament können dann in die medizinische Vorrichtung eingebracht werden. Sodann kann der Aktuator, der die Spritze betätigt, so angesteuert werden, dass die gewünschte Menge an Lösung dem Patienten verabreicht wird. Dies wird beispielsweise dadurch erreicht, dass die Antriebseinheit und ggf. ein von dieser umfasster Antriebskopf, durch Vorschub eines Membrantellers, diesen auf eine Spritzenkolbenplatte drückt und so den Stößel/ Spritzenkolben in den Spritzenkolben drückt. Durch den entstehenden Druck wird die gewünschte Menge der Lösung kontrolliert aus der -Spritzenöffnung einer Spritze zur Verwendung in Spritzenpumpen entlassen.

Es ist eine Vielzahl solcher medizinischen Vorrichtungen, sowie zugehöriger Zubehörartikel im Stand der Technik bekannt. Es besteht jedoch im Allgemeinen das Problem, dass im medizinischen Alltag und gegebenenfalls im Hausgebrauch das richtige Medikament, über den gewünschten Zugang, in der gewünschten Menge und Zeit an den Patienten gelangt, und das medizinische System, umfassend die medizinische Vorrichtung und den medizinischen Zubehörartikel so eingerichtet sein soll, dass eine Verwechslung oder anderweitige versehentliche Falschbedienung möglichst ausgeschlossen wird.

In der Praxis wählt zumeist klinisches Personal einen mit medizinischer Lösung und gegebenenfalls Medikament vorbestückten Zubehörartikel, oder bestückt diesen selbst mit einer Lösung und gegebenenfalls einem Medikament, und setzt sodann den bestückten Zubehörartikeln in die medizinische Vorrichtung ein. Gegebenenfalls kann an einer grafischen Benutzeroberfläche der medizinischen Vorrichtung der ausgewählte Zubehörartikel ausgewählt und bestätigt werden. Modernere Systeme verfügen über eine entsprechende Netzwerkschnittstelle und kommunizieren gegebenenfalls mit einer externen Steuereinrichtung.

Dies ist von besonderer Relevanz, wenn medizinische Systeme, beispielsweise im intensivmedizinischen Bereich, eine Vielzahl von medizinischen Vorrichtungen umfassen, um den Patienten zur selben Zeit eine Vielzahl von verschiedenen Medikamenten und Lösungen, gegebenenfalls über eine Vielzahl von Zugängen bereitzustellen. Dann können hierdurch eine Vielzahl von medizinischen Schläuchen den Patienten umgeben und die Sicherheit vor Fehlern beim Anschluss durch medizinisches Personal kann beeinträchtigt sein.

Wird das falsche Medikament verabreicht, oder eine falsche Förderleistung der medizinischen Vorrichtung gewählt, kann dies zu fatalen Folgen für den Patienten führen. Insbesondere kann die Gabe von Medikamenten in den falschen Patientenzugang unmittelbar hochkritisch sein, oder - insbesondere bei eingestellten geringen Förderraten - kann ein etwaiger vorhandener Alarm erst spät ausgelöst werden, der eine zu geringe Gabe an Medikament oder medizinischer Lösung detektieren würde. Auch die Vermischung von ggf. nicht miteinander kompatiblen Medikamenten und/oder Lösungen kann durch eine fehlende Übersicht über den Leitungsverlauf verschiedener Zubehörartikel und medizinischer Vorrichtungen vorkommen.

Gegenwärtig wird oftmals die Identifizierung der geeigneten Zubehörartikel von Hand durchgeführt und insbesondere durch eine visuelle und manuelle Verfolgung des Schlauchs von der medizinischen Vorrichtung hin zum Patientenzugang. Dabei sind im Stand der Technik verschiedene Lösungen bekannt, die insbesondere eine Markierung der Zubehörartikel vorsehen, um einer Verwechslung beim Bestücken der medizinischen Vorrichtung mit dem Zubehörartikel möglichst entgegenzuwirken.

Aus der US 2021/0008275 A1 ist eine Spritzenpumpe mit angebrachten RFID-Tag bekannt. Die zugehörige Spritzenpumpe weist eine Leseeinheit zum Auslesen der im RFID-Tag gespeicherten Daten auf.

### Kurze Zusammenfassung der Erfindung

Es ist deshalb eine der Erfindung zu Grunde liegende Aufgabe, die oben genannten und weitere Nachteile zu überwinden. Dabei ist es eine weitere der Erfindung zu Grunde liegende Aufgabe, die Sicherheit beim bestimmungsgemäßen Anschluss von medizinischen Lösungen und ggf. Medikamente umfassenden Einmalartikeln, insbesondere von Spritze zur Verwendung in Spritzenpumpen, in eine medizinische Vorrichtung zuverlässig und gleichzeitig kostengünstig zu erhöhen und insbesondere sicherzustellen, dass der jeweilige Einmalartikel der bestimmungsgemäß richtigen medizinischen Vorrichtung zugeordnet wird. Darüber hinaus ist es eine der Erfindung zu Grunde liegende Aufgabe, die bestimmungsgemäße Einstellung der medizinischen Vorrichtung, insbesondere mit Hinblick auf die Abgabe von medizinischer Lösung und ggf. Medikament, das im Zubehörartikel umfasst ist, an den Patienten, zu erleichtern.

Diese und andere Probleme werden durch den Gegenstand der beigefügten unabhängigen Ansprüche gelöst.

### Detailbeschreibung der Erfindung

Die eingangs beschriebenen Aufgaben werden gelöst durch einen medizinischen Einmalartikel für eine medizinische Vorrichtung, insbesondere eine Spritze zur Verwendung in Spritzenpumpen, eine medizinische Vorrichtung, insbesondere einen Spritzenpumpe, zur automatisierten Verabreichung von Medikamenten an einen Patienten, ein System und ein Verfahren zur Erkennungsmerkmal abhängigen, insbesondere personalisierten, Medikamentenverabreichung gemäß den beigefügten unabhängigen Ansprüchen.

Bevorzugte Ausführungsformen können aus den abhängigen Ansprüchen sowie darüber hinaus aus der folgenden Beschreibung entnommen werden, insbesondere umfassend verschiedene Ausführungsformen, wie in den beigefügten Ansprüchen dargelegt und beschrieben.

Der Fachmann wird dabei verstehen, dass jede Ausführungsform, die in der folgenden Beschreibung beschrieben ist, vom Gegenstand der beigefügten Ansprüche erfasst und umfasst wird. Die Ausführungsformen, Merkmale und Kombinationen von Merkmalen, wie hier in Verbindung mit der Erfindung beschrieben, sowie die Kombination von Merkmalen, wie in den beigefügten Ansprüchen angegeben, aber auch jede Kombination von Merkmalen, wie in Verbindung mit den Ausführungsformen erwähnt und beschrieben, gelten als hier offenbart, mindestens jedoch als durch den Fachmann ableitbar.

Insbesondere kann jede Funktion und jede Kombination von Funktionen in den vorliegenden Ausführungsformen beispielsweise in einer unterschiedlichen Kombination beansprucht werden, insbesondere in einer unterschiedlichen Anspruchskategorie, nicht zuletzt, weil der Fachmann erkennen wird, dass jede einzelne Kombination der hier genannten Funktionen dazu geeignet ist, zur Lösung der zugrunde liegenden Aufgabe beizutragen.

Des Weiteren können jede Funktion und jede Kombination von Funktionen in den Ansprüchen und in der untenstehenden Beschreibung unabhängig von dem jeweils beanspruchten Gegenstand verwendet und beansprucht werden, unabhängig von Anspruchsabhängigkeiten und Rückbezügen, sowie unabhängig von der Anspruchskategorie, in der die Funktion beansprucht wird. Zum Beispiel kann in einer willkürlichen Kombination, die aus einem oder mehreren Ansprüchen ausgewählt ist, eine oder mehrere der nachstehend beschriebenen Ausführungsformen und/oder aus den beigefügten Abbildungen vorgestellten Ausführungsformen in Betracht gezogen werden.

Im Rahmen der vorliegenden Anmeldung beziehen sich Begriffe wie "seitlich", "hinten", "vorne", "oben", "unten", "gegenüber", "innen", "außen" oder ähnliche, wie hier verwendet, die die Position eines ersten Objekts relativ zu einem anderen Objekt beschreiben, bevorzugt auf die relative Position eines jeweiligen Teils oder Objekts in Bezug auf seine Position, wenn es vollständig für den vorgesehenen Gebrauch montiert ist. Mit anderen Worten werden diese Begriffe bevorzugt in Bezug auf einen erfindungsgemäßen Einmalartikel oder eine erfindungsgemäße medizinische Vorrichtung mit Hinblick auf die relative Position des jeweiligen Teils oder Objekts in Bezug auf seine Position verwandt, wenn der erfindungsgemäße Einmalartikel vollständig für den vorgesehenen Gebrauch in der erfindungsgemäße medizinische Vorrichtung montiert ist.

Nach einem ersten Aspekt der vorliegenden Erfindung betrifft die Erfindung einen medizinischen Einmalartikel für eine medizinische Vorrichtung, insbesondere Spritzenpumpe, wobei der Einmalartikel bevorzugt eine Spritze zur Verwendung in Spritzenpumpen ist, umfassend ein Körperteil und umfassend ein in einem Teilabschnitt des Körperteils angeordnetes maschinenlesbares Erkennungsmerkmal mit wenigstens einem optisch erfassbaren Code, wobei das maschinenlesbare Erkennungsmerkmal ein Merkmal des Einmalartikels ist oder in dem vorbestimmten Teilabschnitt des Einmalartikels lösbar oder unlösbar, insbesondere werkseitig, angebracht ist, und wobei das Erkennungsmerkmal zumindest bereichsweise umfangsseitig am vorbestimmten Teilabschnitt des Körperteils angeordnet ist.

Die Erfindung geht von der Überlegung aus, dass eine zuverlässige und eindeutige Identifizierung eines medizinischen Einmalartikels vor seiner Verwendung wichtig ist, um eine korrekte Patientenversorgung sicherzustellen. Systeme, bei denen dazu RFID-Tags eingesetzt werden, sind kostenintensiv in der Herstellung und bei Defekt des Chips unbrauchbar.

Wie nunmehr überraschend erkannt wurde, lässt sich eine robuste und zuverlässige und gleichzeitig kostengünstige Erkennung und Identifizierung eines Einmalartikels erzielen durch ein umfangsseitig, d.h. umfangsseitig an einem Außenumfang des Körperteils angebrachtes, optisch erkennbares Erkennungsmerkmal. Das Merkmal umfasst einen optisch erfassbaren Code und keine elektronischen Komponenten wie RFID-Tags, sodass die Erkennung eine hohe Ausfallsicherheit aufweist.

Eine medizinische Vorrichtung gemäß der vorliegenden Erfindung ist bevorzugt eine Spritzenpumpe. Ein medizinischer Einmalartikel gemäß der vorliegenden Erfindung ist bevorzugt eine Spritze zur Verwendung in Spritzenpumpen. Der Einmalartikel kann auch als Schlauch, Behälter oder Kartusche ausgebildet sein.

Der Begriff "Erkennungsmerkmal", wie hierin verwendet, bezeichnet bevorzugt jedwedes Merkmal, das geeignet ist, einen bestimmungsgemäßen Einmalartikel gemäß der vorliegenden Erfindung von anderen zu unterscheiden oder zu identifizieren. In anderen Worten ist ein maschinenlesbares Erkennungsmerkmal ein charakteristisches Element, eine Eigenschaft oder eine Kennzeichnung, die dazu dient, einen bestimmungsgemäßen Einmalartikel gemäß der vorliegenden Erfindung von anderen zu unterscheiden oder zu identifizieren.

Dass das Erkennungsmerkmal zumindest bereichsweise umfangsseitig am Körperteil angeordnet ist, bedeutet bei rotationssymmetrischen Körperteilen, insbesondere zylindrischen Körperteilen wie bei Spritzen, dass das Erkennungsmerkmal in Umfangrichtung auf dem gekrümmten Gehäuse das Erkennungsmerkmal vorhanden ist. Das Erkennungsmerkmal kann dabei 100% des Umfangs, aber auch weniger entlang des Umfangs abdecken. Bevorzugt sind bis zu 160° bzw. 44% des Umfangs nicht von dem oder den Erkennungsmerkmalen abgedeckt.

Bei Körperteilen von beispielsweise Infusionskassetten, die Kanten aufweisen, beispielsweise quaderförmig ausgebildet sind, erstreckt sich das Erkennungsmerkmal umfangsseitig vorzugsweise auf wenigstens drei Flächen.

Ein optisches Merkmal umfasst Merkmale, welche im sichtbaren Licht optisch erkennbar sind, aber auch Merkmale, welche im IR- oder UV-Bereich erkennbar sind. Das optische Merkmal kann so ausgebildet ein, dass es Licht reflektiert und/oder selbständig ausstrahlt (Fluoreszenz).

Der Einmalartikel kann ein- oder mehrteilig gefertigt sein. Dabei ist das maschinenlesbare Erkennungsmerkmal dafür vorgesehen, den Einmalartikel so zu markieren, dass dieser von anderen Einmalartikeln unterschieden bzw. anhand des Erkennungsmerkmals identifiziert werden kann. Dies geschieht insbesondere anhand der Markierung des Einmalartikels mittels des Erkennungsmerkmals an einem vorbestimmten Teilabschnitt des Einmalartikels. Erfindungsgemäß kann das maschinenlesbare Erkennungsmerkmal lösbar oder unlösbar, insbesondere werkseitig, an den Einmalartikel angebracht sein. Das maschinenlesbare Erkennungsmerkmal kann zusätzlich oder alternativ auch ein Merkmal des Einmalartikels selbst sein, beispielsweise ein bedruckter Teilabschnitt der Spritze.

Möglich ist es bspw. bereits werksseitig einen Einmalartikel durch ein permanentes Anbringen des Erkennungsmerkmals identifizierbar zu machen. So kann ein solches Merkmal aufgedruckt sein. Alternativ oder zusätzlich kann der Einmalartikel manuell, bspw. durch Klinikpersonal, mit einem maschinenlesbares Erkennungsmerkmal lösbar oder unlösbar versehen werden. Besonders das nachträgliche Anbringen eines Erkennungsmerkmals vor Anwendung, insbesondere werksseitig, erlaubt eine gezieltes und Benutzer-bestimmtes Individualisieren. Insbesondere ein Spritzenkolben oder eine Spritzenkolbenplatte kann jedoch auch bedruckt und bereits entsprechend werkseitig mit einem Erkennungsmerkmal versehen sein.

Das maschinenlesbare Erkennungsmerkmal, insbesondere, der vorbestimmte Teilabschnitt, ist bevorzugt so ausgewählt, dass dieses von einer medizinischen Vorrichtung, bevorzugt in einem eingesetzten Zustand, eingelesen werden kann.

Der Begriff "in einem eingesetzten Zustand", wie hierin verwendet bezeichnet dabei bevorzugt die korrekte Lage des Einmalartikels in der medizinischen Vorrichtung für den bestimmungsgemäßen Gebrauch, also wenn bspw. die Spritze zur Verwendung in Spritzenpumpen korrekt in den medizinischen Spritzenpumpe eingebracht und gehaltert ist.

In einer bevorzugten Ausführungsform umfasst der optisch erfassbare Code eine Farbkodierung und/oder Strich/Punkt-Kodierung und/oder ein Muster oder einen Barcode oder QR-Code. Die Farbe kann sowohl schwarz-weiß als auch verschiedene Farben umfassen. Die Farbe repräsentiert hierbei eine visuell wahrnehmbare Eigenschaft des Einmalartikels und/oder vorbestimmten Teilabschnitts des Einmalartikels. Ein Muster kann zusätzliche visuelle Elemente beinhalten, die als charakteristisch für die Identifikation dienen. Die Farbkodierung kann beispielsweise, ähnlich wie bei der Farbkodierung von elektronischen Widerständen, mehrere benachbarte Farbstreifen umfassen. Da sich die Struktur Farbstreifen umfangsseitig nicht verändert, ist eine besonders zuverlässige Identifikation es Einmalartikels unabhängig von seiner eingesetzten Lage möglich.

Barcode und QR-Code stellen spezifische optische Codes dar, die durch Scannen oder fotografische Erfassung maschinenlesbar sind und Informationen über den Einmalartikel enthalten. Durch die Verwendung dieser optischen Codes wird eine effiziente und präzise visuelle Identifikation des Einmalartikels ermöglicht, insbesondere in Anwendungen, die auf optischer Erkennung basieren, wie beispielsweise bei der Verwendung von Scannern oder Kameras.

In einer weiteren bevorzugten Ausführungsform umfasst der optisch erfassbare Code in mehrfacher Wiederholung eine Symbol- und/oder Zeichenfolge. Die Zeichenfolge kann dabei das jeweilige Wort, d.h. eine Abfolgen von mehreren Zeichen, enthalten, welches entlang einer Axialrichtung des Köperteils ausgerichtet sind oder das Wort ist jeweils entlang der Umfangsrichtung ausgerichtet.

Die Symbol- und/oder Zeichenfolge umfasst vorteilhafterweise einen Erkennungsdatenabschnitt und einen Anfangsdatenabschnitt und/oder einen Enddatenabschnitt umfasst. Der Erkennungsdatenabschnitt enthält die zur Identifizierung des Einmalartikels notwendigen Daten. Der Anfangsdatenabschnitt signalisiert, dass auf in der Erkennungsdatenabschnitt folgt. Es kann auch alternativ oder zusätzlich ein Enddatenabschnitt vorgesehen sein, welcher das Ende des Erkennungsdatenabschnitts kennzeichnet. Bevorzugt weist der Anfangsdatenabschnitt und/oder der Enddatenabschnitt ausschließlich Symbole und/oder Zeichen auf, welche nicht in dem Erkennungsdatenabschnitt vorkommen. Auf diese Weise können Anfang bzw. Ende des Erkennungsdatenabschnitts eindeutig identifiziert werden. Die Verwendung von eines Anfangs- und/oder Enddatenabschnitts ist besonders vorteilhaft, wenn die Wörter umfangsseitig ausgerichtet sind und wiederholt werden.

In bevorzugten Ausführungsformen umfasst der Code eine Kombination von Farbe, Muster, und Form, wodurch die kodierbare Datenmenge erhöht werden. So können beispielsweise erste Informationen durch eine Farbkodierung und zweite Informationen durch eine Zeichensequenz kodiert werden.

Vorzugsweise ist das Erkennungsmerkmal umfangsseitig mehrmals am Teilabschnitt angeordnet. Dabei sind die Erkennungsmerkmale umfangsseitig bevorzugt durch Lücken, in denen kein Erkennungsmerkmal angeordnet ist, beabstandet. Die Erkennungsmerkmale sind dabei bevorzugt gleichmäßig bzw. symmetrisch, d.h. mit einer vorgegebenen Drehsymmetrie, angeordnet. Das jeweilige Erkennungsmerkmal weist dazu umfangsseitig einen Merkmalsabdeckungsgrad auf und die jeweiliger Lücke weist umfangsseitig einen Lückenabdeckungsgrad auf. Der Lückenabdeckungsgrad ist dabei vorzugweise größer als der Merkmalsanteil bzw. Merkmalsabdeckungsgrad.

Der Merkmalabdeckungsgrad (pro Erkennungsmerkmal) beträgt bevorzugt zwischen 15° und 50° Grad. Bevorzugt sind umfangsseitig wenigstens 15% des Umfangs durch die Erkennungsmerkmale abgedeckt.

In einer beispielhaften und bevorzugten Ausführungsform sind drei Erkennungsmerkmale angeordnet, welche jeweils um 120° versetzt am Umfang angeordnet sind. Dazwischen sind jeweils Lücken gebildet. Bei einem Abdeckungsgrad pro Erkennungsmerkmal von 20° decken die drei Erkennungsmerkmale somit insgesamt bzw. in Summe 60° des Umfangs ab und die Lücken zwischen jeweils zwei Erkennungsmerkmalen decken insgesamt 300° ab (pro Lücke 100°). Bei einer anderen vorteilhaften Ausführung mit einem Merkmalabdeckungsgrad von jeweils 5% und drei Erkennungsmerkmalen sind 15% des Umfangs durch die Erkennungsmerkmale abgedeckt.

Das Körperteil ist vorzugsweise rotationssymmetrisch, insbesondere zylinderförmig, ausgebildet. Ein Beispiel hierfür ist der Spritzenzylinder einer Spritze. In anderen Ausführungen kann das Körperteil boxförmig oder quaderförmig ausgebildet sein.

Das Erkennungsmerkmal ist vorteilhafterweise auf das Körperteil aufgedruckt oder an dem Körperteil angebracht, insbesondere als Banderole, Schrumpfschlauch, Plastikring, Dünnschichtfolien.

In einer bevorzugten Ausführungsform ist der Einmalartikel als Spritzenpumpe ausgebildet, wobei das Körperteil durch den Spritzenzylinder gebildet ist, und wobei das Erkennungsmerkmal in einem vordefinierten Abstand zu den Spritzenzylinderflügeln, insbesondere 0-75 mm, vorteilhafterweise 0,1 mm, angebracht ist. Dadurch, dass der Code umlaufend in einem definierten Abstand von den Spritzenflügeln auf dem Außenumfang des Spritzenzylinders der Spritze angebracht ist, ist er unabhängig vom Drehwinkel der Spritze in der Einlageposition im Spritzenpumpengerät immer zuverlässig erkannt werden kann.

In einem zweiten Aspekt der vorliegenden Erfindung, betrifft die Erfindung eine medizinische Vorrichtung, insbesondere Spritzenpumpe, zur automatisierten Verabreichung von Medikamenten an einen Patienten. Eine erfindungsgemäße medizinische Vorrichtung umfasst
- eine Erkennungseinrichtung zur Identifikation eines zuordenbaren, klassifizierbaren und/oder personalisierbaren oben beschriebenen Einmalartikels, umfassend eine optische Sensoranordnung, die dazu eingerichtet ist, das maschinenlesbare Erkennungsmerkmal des Einmalartikels zu erfassen;
- eine Steuereinrichtung, die mit der Sensoranordnung verbunden ist und auf Basis der erfassten Informationen das maschinenlesbare Erkennungsmerkmal des Einmalartikels identifiziert;
- eine Antriebseinheit, die durch die Steuereinrichtung gesteuert wird, um das Medikament gemäß den erkannten Informationen des Einmalartikels automatisiert an den Patienten zu verabreichen.

Die Steuereinrichtung, die mit der Sensoranordnung verbunden ist, übernimmt die Verarbeitung der erfassten Informationen, um das maschinenlesbare Erkennungsmerkmal des Einmalartikels zu identifizieren. Die Steuereinrichtung fungiert bevorzugt als zentrales Verarbeitungselement und nutzt die von der Sensoranordnung bereitgestellten Daten, um den Einmalartikel eindeutig zu identifizieren. Die Verbindung zwischen der Sensoranordnung und der Steuereinrichtung ermöglicht einen nahtlosen Informationsaustausch, wodurch die erfassten Daten effizient und präzise analysiert werden können. Dabei ist bevorzugt zwischen optischer Sensoranordnung und Steuereinrichtung eine Verarbeitungseinrichtung vorgesehen, die die mit dem Sensor erfassten Informationen verarbeitet, und ggf. dazu gehörige Daten aus einer Speichereinheit abgerufen werden.

Auf Basis dieser Analyse identifiziert die Steuereinrichtung das spezifische Erkennungsmerkmal, das dem zuordenbaren, klassifizierbaren und/oder personalisierbaren Einmalartikel zugeordnet ist. Diese Identifikation bildet die Grundlage für die weiteren Schritte in der Anwendung, sei es in der Medikamentenverabreichung oder einem anderen Kontext, in dem der Einmalartikel eingesetzt wird. Eine Antriebseinheit wird durch die Steuereinrichtung gesteuert, um das Medikament gemäß den erkannten Informationen des Einmalartikels automatisiert an den Patienten zu verabreichen.

In einer bevorzugten Ausführungsform weist die Erkennungseinrichtung eine Beleuchtungseinheit zur Beleuchtung des Erkennungsmerkmals auf.

In einer bevorzugten Ausführungsform ist die Erkennungseinrichtung ausgebildet, ein Erkennungsmerkmal, welches einen mehrfach wiederholten Code aufweist, zu erkennen.

Die Erkennungseinrichtung ist vorteilhafterweise zur Erfassung des Erkennungsmerkmals auf einem Spritzenzylinder eingerichtet. Bei einer vorteilhaft als Spritzenpumpe ausgebildeten medizinischen Vorrichtung ist ein entsprechend optischer Sensor vorteilhaft in der Spritzenmulde und/oder der Innenseite der den Zylinder umschließenden und die Spritzen-mulde verschließende Verschlussklappe angeordnet. Insbesondere sind durch die Fixierung der Spritzenflügel in der Spritzenpumpe ein definierter Abstand entlang der Hauptlängsachse der eingelegten Spritze gegeben, sodass ein entsprechendes Erkennungsmerkmal in einen vorbestimmten Sichtbereich des optischen Sensors gelangt.

Dabei kann der optische Sensor bei geschlossener Klappe vorteilhaft verwendet werden, weil dann eine - dunkle - vorherbestimmbare optische Beleuchtungsumgebung gegeben ist. Insbesondere kann eine oben genannte Beleuchtungseinheit vorgesehen sein, beispielsweise eine Leuchtdiode oder LED, die den Sichtbereich der Kamera und den den Code umfassenden Mantelteil des Spritzenzylinders definiert beleuchtet.

In einer weiteren vorteilhaften Ausführungsform der erfindungsgemäßen medizinischen Vorrichtung weist die medizinische Vorrichtung einen Haltemechanismus, insbesondere Klemm-Mechanismus, zur Halterung des Einmalartikels in der medizinischen Vorrichtung auf. Der Haltemechanismus umfasst dabei vorzugsweise die Erkennungseinrichtung. Bevorzugt ist die Erkennungseinrichtung an einem Haltemechanismus, insbesondere Klemm-Mechanismus, zur Halterung des Einmalartikels in der medizinischen Vorrichtung angebracht und/oder als Teil eines Haltemechanismus, insbesondere Klemm-Mechanismus, zur Halterung des Einmalartikels in der medizinischen Vorrichtung ausgebildet. Dies erlaubt beispielsweise, die Erkennung eines Erkennungsmerkmals, das an einem für die Halterung vorgesehenen Bauteils des Einmalartikels vorgesehen ist.

Nach einem dritten Aspekt der vorliegenden Erfindung, betrifft die Erfindung ein System zur Erkennungsmerkmal-abhängigen, insbesondere personalisierten, Medikamentenverabreichung, umfassend eine Speichereinheit, die bevorzugt Informationen zu Komponenten des Systems und/oder eine Medikamentendatenbank, in welcher Daten zu Medikamenten hinterlegt sind und/oder eine Patientendatenbank, in welcher Daten zum Patienten hinterlegt sind umfasst.

Das System umfasst eine oben beschriebene Vorrichtung, umfassend eine Kommunikationsschnittstelle zur Übermittlung der erfassten Informationen sowie einen oben beschriebenen medizinischen Einmalartikel für die medizinische Vorrichtung, insbesondere Spritzenpumpe, wobei der Einmalartikel insbesondere eine Spritze zur Verwendung in Spritzenpumpen ist, oder alternativ oder in Kombination dazu eine Leseeinrichtung mit einer Erkennungseinrichtung zur Identifikation eines oben beschriebenen zuordenbaren, klassifizierbaren und/oder personalisierbaren Einmalartikels, umfassend eine optische Sensoranordnung, die dazu eingerichtet ist ein maschinenlesbares Erkennungsmerkmal des Einmalartikels zu erfassen.

Die Erkennungseinrichtung und der Einmalartikel sind dabei bevorzugt derart konfiguriert, dass die Erkennungseinrichtung ausgebildet ist bzw. ein entsprechendes Sensorfenster aufweist, welches die Ausdehnung von wenigstens zwei Erkennungselementen hat, sodass immer mindestens ein Erkennungselement erkannt werden kann.

Die Speichereinheit kann lokal in der Vorrichtung und/oder außerhalb der Vorrichtung vorgesehen sein, wobei eine außerhalb der Vorrichtung vorgesehen Speichereinheit insbesondere eine Netz-, Server- und/oder Cloud-basierte Speichereinheit umfasst.

Die Kommunikationsschnittstelle ist dabei bevorzugt vorgesehen und eingerichtet, die erfassten Informationen an eine Speichereinheit zu übermitteln und/oder Daten aus der Speichereinheit abzurufen.

Die Speichereinheit umfasst bevorzugt Informationen zu Komponenten des Systems, bspw. Informationen zur medizinischen Vorrichtung, z.B. zur Pumpe, Pumpenleistung, Pumpen-Spezifikation oder weitere Vorrichtungsmerkmale. Gleichzeitig oder alternativ umfasst die Speichereinheit bevorzugt eine Patientendatenbank, in welcher Daten zum Patienten hinterlegt sind, bspw. eine Patienten-ID, Behandlungsschemata und Medikationsdetails, oder ähnliches. Es können auch weitere Informationen in der Patientendatenbank hinterlegt sein, insbesondere persönliche und/oder gesundheitsbezogene Patientendaten, wie Alter, Gewicht, Name, usw.

Gleichzeitig oder alternativ umfasst die Speichereinheit bevorzugt eine Medikamentendatenbank, in welcher Daten zu Medikamenten hinterlegt sind, bspw. Dosierungsschemata, Ablaufdatum, oder ähnliches.

Nach einem vierten Aspekt der vorliegenden Erfindung, betrifft die Erfindung ein Verfahren zur Erkennungsmerkmal abhängigen, insbesondere personalisierten, Medikamentenverabreichung in einem medizinischen System, insbesondere für Spritzenpumpen, umfassend die Schritte:
- Bereitstellen einer oben beschriebenen medizinischen Vorrichtung und/oder eines oben beschriebenen Systems,
- Bereitstellen eines oben beschriebenen Einmalartikels für oder Einbringen eines oben beschriebenen Einmalartikels in die medizinische Vorrichtung,
- Erkennen des Einmalartikels bevorzugt mittels eine Erkennungseinrichtung der medizinischen Vorrichtung, mittels des Erkennungsmerkmals des Einmalartikels;
- Identifizieren des Erkennungsmerkmals des Einmalartikels auf Basis der erfassten Informationen;
- Steuern einer Antriebseinheit, bevorzugt mittels einer Steuereinrichtung der medizinischen Vorrichtung, zur automatisierten Verabreichung eines Medikaments gemäß erkannten Informationen des Einmalartikels.

Die oben angegebenen Schritte müssen nicht zwangsläufig in der gegebenen Reihenfolge durchgeführt werden. Die bereitgestellten Schritte können in jeder anderen geeigneten Reihenfolge durchgeführt werden. Die oben angegebene Reihenfolge kann jedoch für bestimmte Ausführungsformen des Verfahrens gelten.

Es wird unmittelbar von einer fachkundigen Person erkannt, dass eine Eigenschaft, Ausführungsform, Wirkung oder Vorteil, die hier in Verbindung mit dem erfinderischen medizinischen Einmalartikel für eine medizinische Vorrichtung, insbesondere eine Spritze zur Verwendung in einer Spritzenpumpe, der medizinischen Vorrichtung, insbesondere einen Spritzenpumpe, zur automatisierten Verabreichung von Medikamenten an einen Patienten und dem System zur Erkennungsmerkmal abhängigen, insbesondere personalisierten, Medikamentenverabreichung, beschrieben wird, auch eine Eigenschaft, Ausführungsform, Wirkung oder Vorteil des erfinderischen Verfahrens sein kann, und umgekehrt.

Alle beschriebenen Ausführungsformen der vorliegenden Erfindung haben den Vorteil, dass sowohl kurzfristig als auch mittel- bis langfristig signifikante Verbesserungen gegenüber den im Stand der Technik bekannten Lösungen bestehen. Das Erkennungsmerkmal erhöht besonders vorteilhaft die Sicherheit bei der Auswahl und Anwendung von Medizinprodukten durch die Möglichkeit der Vorparametrisierung.

Zudem geht mit der vorliegenden Erfindung nur ein relativ geringer Kostenmehraufwand für den Einmalartikel einher. Die vorliegende Erfindung ermöglicht ebenfalls eine Integration in verschiedene Anwendungsbereiche wie Homecare, da die Vermeidung einer Fehlzuordnung von Medikament zu Patienten vermieden wird, und somit ungeschultes Personal leichter die vorliegende Erfindung anwenden kann. Weiterhin können vordefinierte Informationen leichter übertragen werden, bspw. die Übertragung von Therapiemustern auf verschiedene medizinische Vorrichtungen erlauben, die medizinische Vorrichtung leicht zu tauschen. Die mögliche Cloud-Anbindung gewährleistet zudem eine sichere und effiziente Datenübertragung, unabhängig von der Standortentfernung. Insgesamt tragen die Sicherheitsmerkmale dazu bei, eine korrekte Identifikation von personalisierten Einmalartikeln sicherzustellen und erhöhen somit die Patientensicherheit.

Die umlaufende Kodierung des Einmalartikels spielt hier eine besondere Rolle, da damit eine positionsunabhängige Erkennung entlang des rotationssymmetrischen Körpers des Einmalartikels möglich ist. Aufgrund der umlaufenden Ablesbarkeit ist die Orientierung von Lesegerät zu Codierung frei zur Rotation des Einmalartikels. Durch die zuverlässige Identifizierung des Erkennungsmerkmals werden die Sicherheit bei der Anwendungsauswahl erhöht und eine Vorparametrisierung ermöglicht.

Die vorgeschlagene Kennzeichnung des Einmalartikels mit dem Erkennungsmerkmal führt zu einem geringen Kostenmehraufwand für dem Einmalartikel, insbesondere, wenn von vorneherein eine Bedruckung des Einmalartikels vorgesehen ist. Das alternative Anbringen von umlaufenden Banderolen oder Aufklebern ist eine kostengünstige Möglichkeit, nachträglich das Erkennungsmerkmal am Körperteil des Einmalartikels anzubringen.

Eine Kombination von Farbe, Muster, und Form im optischen Code erhöht die kodierbare Datenmenge. Bei der Erkennung des Erkennungsmerkmals ist eine Sensorfusion, z.B. mit einer Durchmesserbestimmung des Spritzenkolbens, möglich, wodurch ein Wegfall der Spritzenerkennung durch Spritzenkörperdurchmesser ermöglicht wird.

### Beschreibung der Figuren

Die vorliegende Erfindung wird im Folgenden unter Bezugnahme auf die Zeichnungen näher erläutert, aus denen weitere Merkmale, Ausführungsformen und Vorteile entnommen werden können. Darin zeigen
FIG. 1, 2, 3, 4, 5 und 6 schematische Darstellung von als Spritze ausgebildeten erfindungsgemäßen Einmalartikeln in bevorzugten Ausführungsformen, und
FIG. 7 eine als Spritzenpumpe ausgebildete erfindungsgemäße medizinische Vorrichtung in einer bevorzugten Ausführungsform.

Die in der Beschreibung, den Ansprüchen, Beispielen und/oder den Figuren offengelegten Merkmale der vorliegenden Erfindung können sowohl einzeln als auch in beliebiger Kombination dazu dienen, die Erfindung in verschiedenen Formen zu realisieren.

In den in den Figuren gezeigten Ausführungsformen werden Elemente, die in Funktion ähnlich oder identisch sind, mit gleichen Bezugszeichen bezeichnet. Es sei darauf hingewiesen, dass die Figuren möglicherweise nicht maßstabsgetreu zueinander sind.

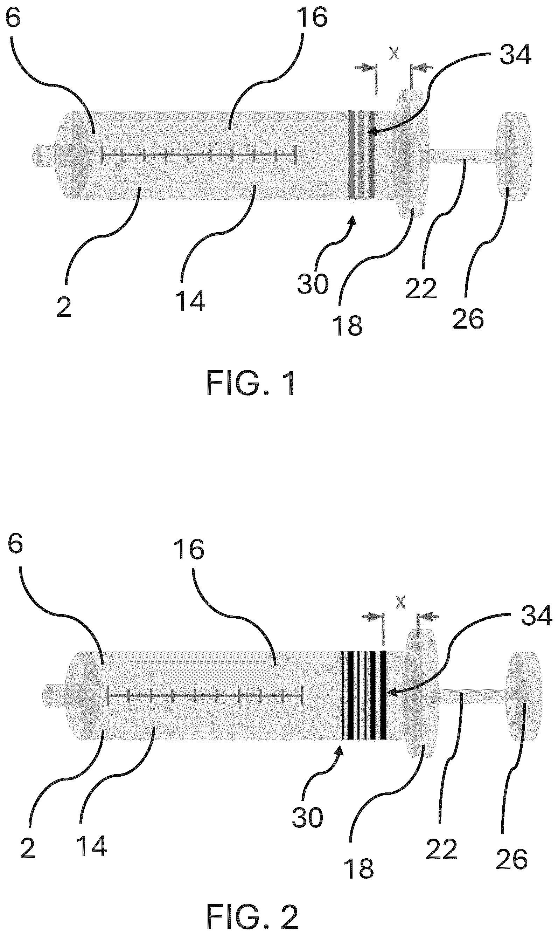

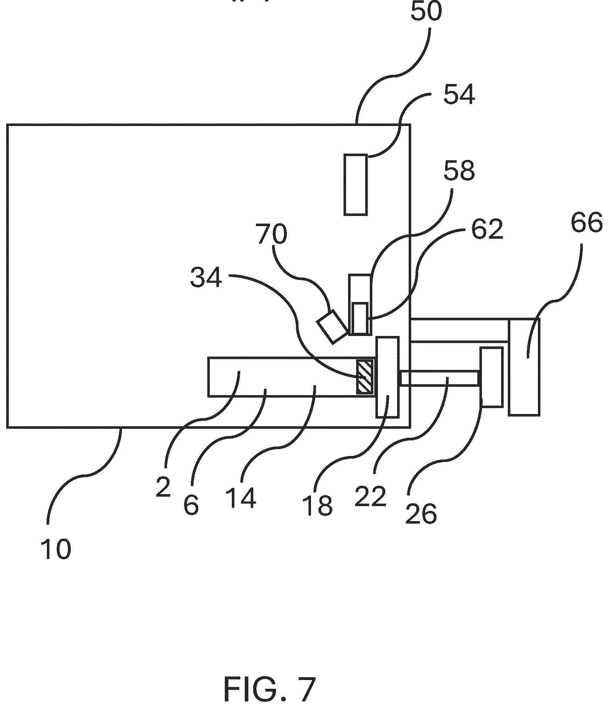

In FIG. 1 ist ein Einmalartikel 2 in einer ersten bevorzugten Ausführungsform dargestellt, welcher vorliegend als Spritze 6 zur Verwendung in einer Spritzenpumpe 10 (siehe FIG. 7) ausgebildet ist.

Die Spritze 6 umfasst einen Spritzenzylinder 14, welcher ein Körperteil 16 des Einmalartikels 2 ist und in dem das zu verabreichende Medikament beinhaltet ist. Zum manuellen Gebrauch sind üblicherweise an dem Spritzenzylinder 14 Spitzenzylinderflügel 18 angeformt, um ein manuelles Verabreichen eines Medikaments mit dem Spritzenpumpe 2, bspw. mit aufgesetzter Kanüle zu erleichtern. Diese Spitzenzylinderflügel 18 dienen zusätzlich auch dazu, den Spritzenzylinder 14 in der Spritzenpumpe 10 zu halten und einzuklemmen.

In den Spritzenzylinder 14 ist ein in Form eines Spritzenkolbens 22 eingeführt, an dessen Ende eine Spritzenkolbenplatte 26 angeformt ist. Wird bestimmungsgemäß die Spritzenkolbenplatte 26 gedrückt, wird hierdurch der Spritzenkolben 22 in dem Spritzenzylinder 14 bewegt und das Medikament aus der Spritze 6 zur Verwendung der Spritzenpumpe 10 gedrückt

An einem vorbestimmen Teilabschnitt 30 des Körperteils 16 ist ein Erkennungsmerkmal 34 angeordnet, welches vorliegend auf den Spritzenzylinder 14 aufgedruckt ist. Das Erkennungsmerkmal 34 ist vorliegend als dreistreifiger Farbcode ausgebildet mit drei benachbarten Farbstreifen, wobei jeder der drei Farbstreifen in dem Teilabschnitt 30 umfangsseitig um den Spritzenzylinder 14 umläuft. Durch die Verwendung von Farbstreifen kann im Sinne einer Farbkodierung, wie sie bei elektronischen Widerständen verwendet wird, eine eindeutige Identifikation des Einmalartikels 2 erfolgen. Es können auch weniger oder mehr Farbstreifen verwendet werden.

Durch das umgangsseitige Umlaufen des Erkennungsmerkmals 34, vorliegend der Farbstreifen, wird eine rotationsunabhängige Erkennung der Spritze 6 möglich, da unabhängig vom Drehwinkel des Spritzenzylinders 6 bei in die Spritzenpumpe 10 eingelegter Spritze 6 das Erkennungsmerkmal 34 durch die Erkennungseinrichtung 58 erkannt werden kann.

Das Erkennungsmerkmal 34 weist einen definierten Abstand X zu den Spritzenzylinderflügeln 18 auf. Da die Spritzenzylinderflügel 18 in der Spritzenpumpe 10 zur Fixierung der Spritze 6 und damit der Fixierung der räumlichen Position des Spritzenzylinders 14 dienen, ist damit eine definierte relative Lage des Erkennungsmerkmals 34 zu einer Erkennungseinrichtung 58 (siehe FIG. 7) der Spritzenpumpe 10 möglich. Der Abstand X ist beispielhaft vorliegend 3 mm.

In FIG. 2 ist ein als Spritze 6 ausgebildeter Einmalartikel 2 in einer weiteren bevorzugten Ausführungsform dargestellt. Das Erkennungsmerkmal 34 ist in diesem Ausführungsbeispiel als Barcode ausgebildet, wobei die Striche des Barcodes in dem Teilabschnitt 30 umfangsseitig um den Spritzenzylinder 14 laufen.

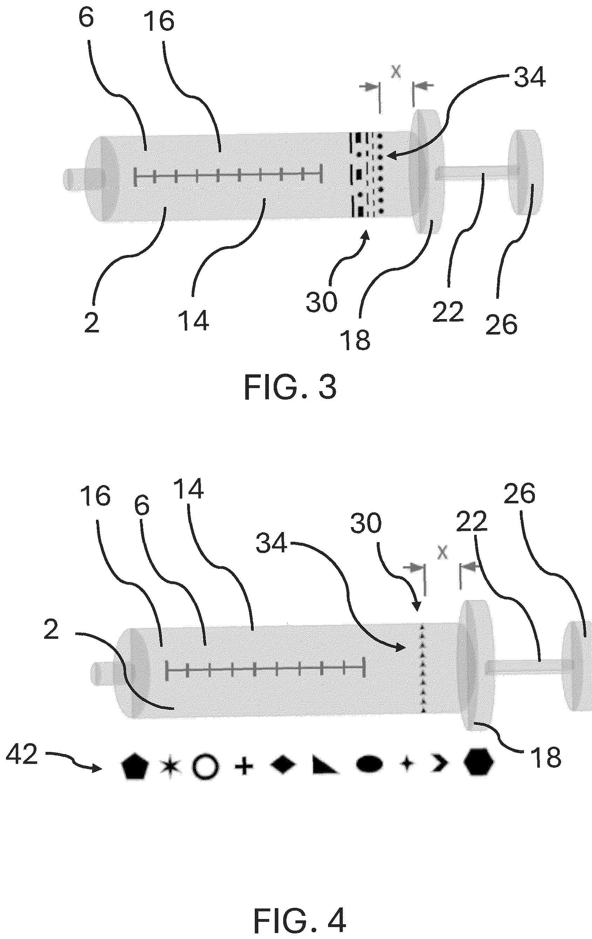

In FIG. 3 ist ein als Spritze 6 ausgebildeter Einmalartikel 2 in einer weiteren bevorzugten Ausführungsform dargestellt. Das Erkennungsmerkmal 34 ist in diesem Ausführungsbeispiel als Strich-Punkt-Kodierung ausgebildet, wobei die Striche und Punkte der Strich-Punkt-Kodierung in dem Teilabschnitt 30 umfangsseitig um den Spritzenzylinder 14 laufen. Die Strich-Punkt-Kodierung kann nach dem Leitungskodierungsprinzip ausgebildet sein.

In FIG. 4 ist ein als Spritze 6 ausgebildeter Einmalartikel 2 in einer weiteren bevorzugten Ausführungsform dargestellt. Das Erkennungsmerkmal 34 ist in diesem Ausführungsbeispiel als Musterkodierung bzw. Formenkodierung ausgebildet, wobei eine Sequenz von gefüllten Dreiecken in dem Teilabschnitt 30 umfangsseitig um den Spritzenzylinder 14 läuft. Statt Dreiecken können auch andere Symbole verwendet werden. In FIG. 4 ist auch eine Musterkodierung 42 dargestellt, welche beispielhaft geeignete Symbole zeigt, beispielsweise Vielecke, Dreieck, Kreis, Raute, Punkt, Trapez, Stern, Kreuz, Vieleck, etc.

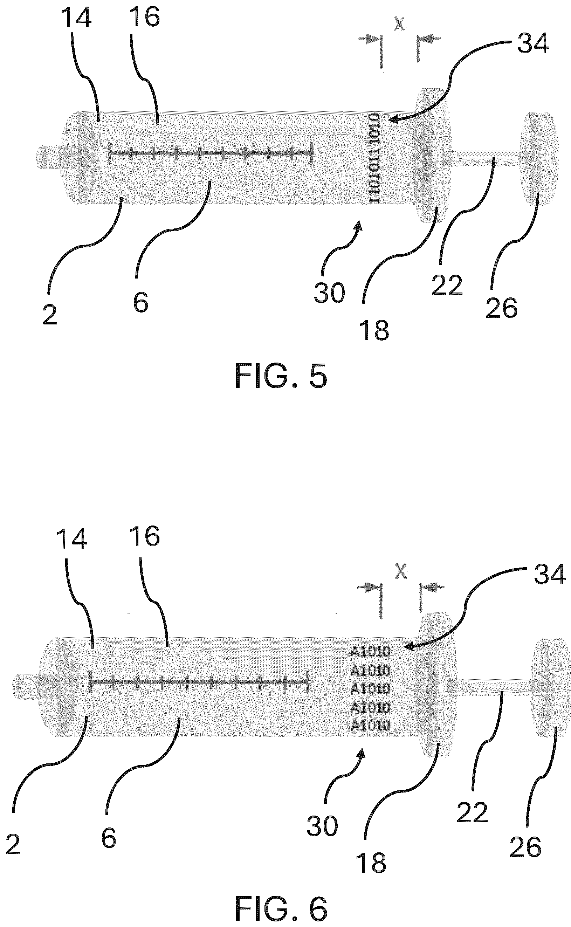

In FIG. 5 ein als Spritze 6 ausgebildeter Einmalartikel 2 in einer weiteren bevorzugten Ausführungsform dargestellt. Das Erkennungsmerkmal 34 ist in diesem Ausführungsbeispiel als umfangsseitig umlaufende alphanumerische Zeichenkette ausgebildet, wobei die Zeichenkette entlang des Umfangs ausgerichtet ist. Die Zeichenkette kann genau einmal entlang des Umfangs angeordnet sein oder mehrfach, d.h. wiederholt. Insbesondere eine Wiederholung entlang des Umfangs kann eine rotationsunabhängige Erkennung des Erkennungsmerkmals 34 ermöglichen.

In FIG. 6 ist ein als Spritze 6 ausgebildeter Einmalartikel 2 in einer weiteren bevorzugten Ausführungsform dargestellt. Das Erkennungsmerkmal 34 ist in diesem Ausführungsbeispiel als sich umfangsseitig wiederholende umlaufende alphanumerische Zeichenkette ausgebildet, wobei die Zeichenkette in Axialrichtung ausgerichtet ist.

In FIG. 7 ist sehr schematisch eine medizinische Vorrichtung 50, welche als Spritzenpumpe 10 ausgebildet ist, dargestellt. Die Anordnung der einzelnen Komponenten ist dabei sehr schematisch dargestellt und entspricht nicht notwendigerweise der bevorzugten tatsächlichen räumlichen Anordnung. Der als Spritze 6 ausgebildete Einmalartikel 2 ist in die Spritzenpumpe 10 eingesetzt. Die Spritzenpumpe 10 umfasst eine Steuereinrichtung 54, welche mit einer Erkennungseinrichtung 58 mit einer optischen Sensoranordnung 62 verbunden ist und welche auf Basis der erfassten Informationen das maschinenlesbare Erkennungsmerkmal 2 der Spritze 6 identifiziert. Die Spritzenpumpe 6 umfasst weiter eine Antriebseinheit 66, die durch die Steuereinrichtung 54 gesteuert wird, um das Medikament gemäß den erkannten Informationen des Einmalartikels 2 automatisiert an den Patienten zu verabreichen.

Die Spritzenpumpe 10 umfasst auch einen Haltemechanismus (nicht dargestellt), insbesondere einen Klemm-Mechanismus, der die Spritze 6 in der Spritzenpumpe 10 hält. Insbesondere werden die Spritzenzylinderflügel 18 eingeklemmt. Das Erkennungsmerkmal 34 ist in einem definierten Abstand X von den Spritzenzylinderflügeln 18 auf dem Spritzenzylinder 14 angebracht. Die Erkennungseinheit 58 ist ausgebildet, in diesem Bereich das Erfassungsmerkmal 34 zu erfassen und zu erkennen. Die Spritzenpumpe 10 weist eine Beleuchtungseinheit 70 zur Beleuchtung des Erkennungsmerkmals 34 auf, wodurch die optische Erkennung erleichtert wird.

Die optische Sensoranordnung umfasst bevorzugt eine Kamera, optional mit einer kleinen Leuchtquelle (z.B. Diode), und ist bevorzugt in der Mulde oder der Klappe der Spritzenpumpe 10 angeordnet. Diese Kamera erfasst den optischen Code und übermittelt die Informationen an die Steuereinrichtung 54 der Pumpe. Die Leuchtquelle der Beleuchtungseinheit 70 sorgt für eine gleichmäßige Ausleuchtung des Codes, wodurch die Erkennungsgenauigkeit erhöht wird.

### Bezugszeichenliste

- 2: Einmalartikel
- 6: Spritze
- 10: Spritzenpumpe
- 14: Spritzenzylinder
- 16: Körperteil
- 18: Spitzenzylinderflügel
- 22: Spritzenkolben
- 26: Spritzenkolbenplatte
- 30: Teilabschnitt
- 34: Erkennungsmerkmal
- 42: Mustersequenz
- 50: medizinische Vorrichtung
- 54: Steuereinrichtung
- 58: Erkennungseinrichtung
- 62: optische Sensoranordnung
- 66: Antriebseinheit
- 70: Beleuchtungseinheit

- X: Abstand

## Patentansprüche

1. Medizinischer Einmalartikel (2) für eine medizinische Vorrichtung (50), insbesondere Spritzenpumpe (10), wobei der Einmalartikel (2) bevorzugt eine Spritze (6) zur Verwendung in Spritzenpumpen (10) ist, umfassend ein Körperteil (16) und umfassend ein in einem Teilabschnitt (30) des Körperteils (16) angeordnetes maschinenlesbares Erkennungsmerkmal (34) mit wenigstens einem optisch erfassbaren Code, wobei das maschinenlesbare Erkennungsmerkmal (34) ein Merkmal des Einmalartikels (2) ist oder in dem vorbestimmten Teilabschnitt (30) des Einmalartikels (2) lösbar oder unlösbar, insbesondere werkseitig, angebracht ist, und wobei das Erkennungsmerkmal (34) zumindest bereichsweise umfangsseitig am vorbestimmten Teilabschnitt (30) des Körperteils (16) angeordnet ist.

2. Einmalartikel (2) nach Anspruch 1, wobei der optisch erfassbare Code eine Farbkodierung und/oder Strich/Punkt-Kodierung und/oder ein Muster oder einen Barcode oder QR-Code umfasst.

3. Einmalartikel (2) nach Anspruch 1 oder 2, wobei der optisch erfassbare Code in mehrfacher Wiederholung eine Symbol- und/oder Zeichenfolge umfasst.

4. Einmalartikel (2) nach Anspruch 3, wobei die Symbol- und/oder Zeichenfolge einen Erkennungsdatenabschnitt und einen Anfangsdatenabschnitt und/oder einen Enddatenabschnitt umfasst.

5. Einmalartikel (2) nach einem der Ansprüche 1 bis 4, wobei das Erkennungsmerkmal (34) umfangsseitig mehrmals am Teilabschnitt (30) angeordnet ist.

6. Einmalartikel (2) nach einem der Ansprüche 1 bis 5, wobei das Körperteil (16) rotationssymmetrisch ausgebildet ist.

7. Einmalartikel (2) nach einem der vorherigen Ansprüche, wobei das Erkennungsmerkmal (34) auf das Körperteil (16) aufgedruckt ist oder wobei das Erkennungsmerkmal (34) an dem Körperteil (16) angebracht ist, insbesondere als Banderole, Schrumpfschlauch, Plastikring, Dünnschichtfolien.

8. Einmalartikel (2) nach einem der vorherigen Ansprüche, welches als Spritzenpumpe (6) ausgebildet ist, wobei das Körperteil (16) durch den Spritzenzylinder (14) gebildet ist, und wobei das Erkennungsmerkmal (34) in einem vordefinierten Abstand, insbesondere 0 bis 75 mm, zu den Spritzenzylinderflügeln (18) angebracht ist.

9. Medizinische Vorrichtung (50), insbesondere Spritzenpumpe (10), zur automatisierten Verabreichung von Medikamenten an einen Patienten, umfassend:
- eine Erkennungseinrichtung (58) zur Identifikation eines zuordenbaren, klassifizierbaren und/oder personalisierbaren Einmalartikels (2) nach einem der vorherigen Ansprüche, umfassend eine optische Sensoranordnung (62), die dazu eingerichtet ist ein maschinenlesbares Erkennungsmerkmal (34) des Einmalartikels (2) zu erfassen;
- eine Steuereinrichtung (54), die mit der Sensoranordnung (62) verbunden ist und auf Basis der erfassten Informationen das maschinenlesbare Erkennungsmerkmal (34) des Einmalartikels (2) identifiziert;
- eine Antriebseinheit (66), die durch die Steuereinrichtung (54) gesteuert wird, um das Medikament gemäß den erkannten Informationen des Einmalartikels (2) automatisiert an den Patienten zu verabreichen.

10. Medizinische Vorrichtung (50) nach Anspruch 9, wobei die Erkennungseinrichtung (58) eine Beleuchtungseinheit (70) zur Beleuchtung des Erkennungsmerkmals (34) aufweist.

11. Medizinische Vorrichtung (50) nach Anspruch 9 oder 10, wobei die Erkennungseinrichtung (58) ausgebildet ist, einen mehrfach wiederholten Code in dem Erkennungsmerkmal (34) zu erkennen.

12. Medizinische Vorrichtung (50) nach einem der Ansprüche 9 bis 11, die als Spritzenpumpe (10) ausgebildet ist.

13. Medizinische Vorrichtung (50) nach Anspruch 11, wobei die Erkennungseinrichtung (58) zur Erfassung des Erkennungsmerkmals (34) auf einem Spritzenzylinder (14) eingerichtet ist.

14. System zur Erkennungsmerkmal-abhängigen, insbesondere personalisierten, Medikamentenverabreichung, umfassend:
- eine Speichereinheit, die bevorzugt Informationen zu Komponenten des Systems und/oder eine Medikamentendatenbank, in welcher Daten zu Medikamenten hinterlegt sind und/oder eine Patientendatenbank, in welcher Daten zum Patienten hinterlegt sind umfasst;
- eine medizinische Vorrichtung (50) nach einem der Ansprüche 9 bis 13 oder ein Lesegerät, umfassend eine Kommunikationsschnittstelle zur Übermittlung der erfassten Informationen und/oder eine Leseeinrichtung mit einer Erkennungseinrichtung (58) zur Identifikation eines zuordenbaren, klassifizierbaren und/oder personalisierbaren Einmalartikels (2) nach einem der Ansprüche 1 bis 8, umfassend eine optische Sensoranordnung (62), die dazu eingerichtet ist ein maschinenlesbares Erkennungsmerkmal (34) des Einmalartikels (2) zu erfassen;
- einen medizinischer Einmalartikel (2) nach eine der Ansprüche 1 bis 8 für eine medizinische Vorrichtung (50) insbesondere Spritzenpumpe (10), wobei der Einmalartikel (2) insbesondere eine Spritze (6) zur Verwendung in Spritzenpumpen (10) ausgebildet ist.

15. Verfahren zur Erkennungsmerkmal abhängigen, insbesondere personalisierten, Medikamentenverabreichung in einem medizinischen System, insbesondere für Spritzenpumpen (10), umfassend die Schritte:
- Bereitstellen einer medizinischen Vorrichtung (50) nach einem der Ansprüche 9 bis 13 und/oder eines Systems nach Anspruch 14,
- Bereitstellen eines oben beschriebenen Einmalartikels für oder Einbringen eines Einmalartikels (2) nach einem der Ansprüche 1 bis 8 in die medizinische Vorrichtung (50),
- Erkennen des Einmalartikels (2), bevorzugt mittels einer Erkennungseinrichtung (58) der medizinischen Vorrichtung (50), mittels des Erkennungsmerkmals (34) des Einmalartikels (2);
- Identifizieren des Erkennungsmerkmals (34) des Einmalartikels (2) auf Basis der erfassten Informationen;
- Steuern einer Antriebseinheit (66), bevorzugt mittels einer Steuereinrichtung (54) der medizinischen Vorrichtung (10), zur automatisierten Verabreichung eines Medikaments gemäß erkannten Informationen des Einmalartikels (2).
